# EUROPEAN PATENT SPECIFICATION

(11) **EP 3 787 801 B1**
(45) Date of publication and mention of the grant of the patent: **12.07.2023**
(21) Application number: 18917218.2
(22) Date of filing: 04.05.2018
(51) Int. Cl.: B05B 11/00, A61M 11/00

(54) **MICROSTRUCTURED NOZZLE**
MIKROSTRUKTURIERTE DÜSE
BUSE MICROSTRUCTURÉE

(43) Date of publication of application: 10.03.2021
(73) Proprietor: Microbase Technology Corp., Taoyuan City 33464 (TW)
(72) Inventor: LIN, Yi-ting, Taoyuan City, Taiwan 33464 (TW); CHEN, Po-chuan, Taoyuan City, Taiwan 33464 (TW)
(74) Representative: Betten & Resch
(86) International application number: PCT/CN2018/085665
(87) International publication number: WO 2019/210515

(56) References cited:
- EP-A1- 1 493 492
- WO-A1-97/12683
- WO-A1-2005/014175
- CN-A- 1 271 296
- CN-A- 1 809 424
- CN-A- 1 921 949
- TW-A- 201 817 497
- US-B2- 9 283 333

## Description

### FIELD

The present disclosure relates to a microstructured passage module and more particularly to a microstructured passage module for an aerosol generator.

### BACKGROUND

Aerosolizer, also known as nebulizer or atomizer, is used to deliver medication to patients for inhalation. Particularly, liquid medicament is broken down into aerosol having fine particles/droplets for easier and more efficient inhalation and absorption. The particle size may be adjusted depending on different respiratory conditions, such as Chronic Obstructive Pulmonary Disease (COPD) or asthma, or depending on the requirement of the liquid medicament itself. The ability to receive the same precise amount of medication in each treatment is also very important for patients. In other words, a good aerosolizer should be able to deliver a precise dosage of medication having a fixed average particle size, a certain range of MMAD (Mass Median Aerodynamic Diameter), and at certain spray duration in every operation to reduce waste and risks of overdosing.

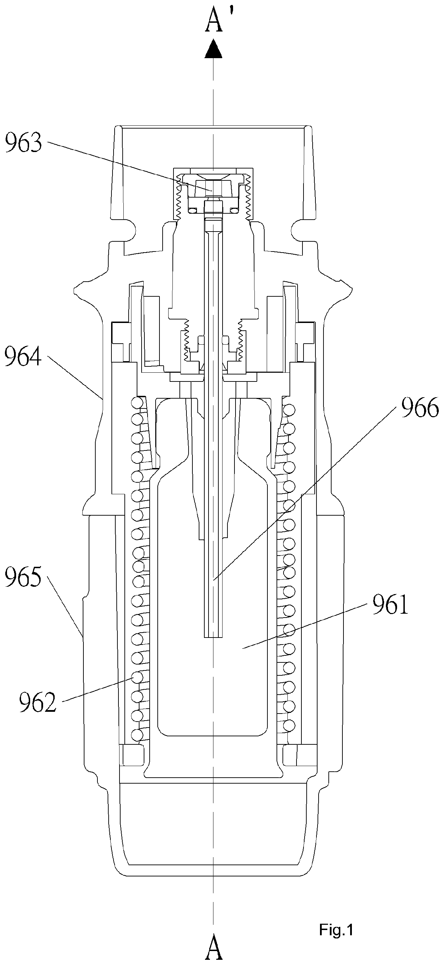

Referring to FIG. 1, an exemplary aerosolizer includes an upper housing 964, a lower housing 965, a nozzle 963, a tube 966, a biasing element 962 and a storage container 961. During preparation, the biasing element 962, such as a spring, is tensioned by the relative movement of the upper housing 964 and the lower housing 965. Meanwhile, a fixed amount liquid medicament (not shown) is drawn from the storage container 961 by the tube 966 and to the nozzle 963, ready to be aerosolized. When the aerosolizer is actuated, a force generated by the un-tensioned biasing element 962 pushes the fixed amount of liquid medicament towards and through the nozzle 963, thereby creating the aerosol for inhalation. Another exemplary aerosolizer and the operation mechanism thereof can be referenced to the disclosure in US5964416 (US Patent Application Number: 08/726,219).

As shown in FIG. 1, pressurized liquid medicament travels in the direction from A to A', i.e., from a high pressure point to a low pressure point. Liquid medicament is drawn and forced into the nozzle 963, through which aerosol is generated and exited out. During aerosolization, it is crucial that proper seal is maintained between the components inside the aerosolizer. Otherwise, the resulting aerosolization effect may be compromised. For example, a leak at the nozzle 963 may lead to pressure loss, which can result in delivery of inaccurate dosage or undesired aerosol particle size. The foregoing might affect the MMAD and the spray duration thereof. To achieve proper seal, components of the aerosolizer must be manufactured and assembled with caution and precision. However, due to the miniature size of the components, usually in the scale of millimeters or less, achieving proper seal during manufacturing tends to be difficult and costly. Moreover, miniature components of different geometric shapes may be more prone to wear and tear in a high-pressure (usually between 5 and 50 MPa, which is about 50 to 500 bar) environment.

In another aspect, the nozzle 963 plays a pivotal role in whether the pressurized liquid medicament can be aerosolized into fine particles/droplets and exit the aerosolizer at a certain speed. As shown in FIG. 1, the pressurized liquid medicament travels through the central connecting tube to the nozzle 963. The pressurized liquid medicament generally flows into the nozzle 963 at a high speed. The nozzle 963 serves to filter and decrease the flow speed of the liquid medicament in a controlled manner such that precise dosage of medicament can be aerosolized into the desired aerosol form. The foregoing may be achieved through specifically configured internal structure of the nozzle 963. Improper design of the nozzle 963 may lead to blockage to the entire aerosolization process, which may shorten the life of the aerosolizer or affect dosage accuracy.

A typical nozzle used in an aerosolizer includes multiple elements with different geometric shapes. For example, some elements with a particular shape, e.g., elongated projections, are used as filters. Some other elements with a different shape, e.g., cylindrical projections, are used to structure a guiding system to control the liquid flow in the nozzle. In short, a nozzle used in the relevant art requires the combination and interaction of multiple elements having different structural and/or functional characteristics in order to achieve the desired aerosolization effect. However, due to the miniature size of the nozzle, fluid control therein is not easy. The structure, dimension and arrangement of the elements in the nozzle need to be carefully implemented to make the nozzle effective. As a result, the costs for the design and manufacture of the nozzle tends to be high.

Document EP1493492A1 relates to a micro structured high pressure nozzle unit with built in filter function for high pressure atomizers for nebulizing fluids.

The present disclosure aims to provide a nozzle structure with less complicated structure, design and arrangement. The resulting nozzle will improve the overall aerosolization quality and efficiency, while the cost for manufacturing such nozzle is reduced. Accordingly, patients can enjoy a more affordable treatment solution.

### SUMMARY

The present invention is defined in independent claims 1 and 9. Embodiments are defined in the dependent claims. There is provided a microstructured passage module for an aerosol generator. The module includes a plate overlaid by a cover thus forming a compartment, an entrance for a liquid and an exit. The plate further includes a filtering structure. An exemplary filtering structure includes walls, pillars, protrusions, and combination thereof. In some embodiments, the plate includes a plurality of walls parallel to each other over its entire width so as to define a plurality of passages threrebetween. The walls are arranged along a flow direction, which is substantially perpendicular to the entrance. In certain embodiments, a plurality of pillars protruding from the plate are evenly distributed in at least a section of the passages. In yet some other embodiments, the wall could be configured continuously or un-continuously. A column is disposed proximate to the exit and blocks a substantial part thereof, leaving longitudinal aisles for the liquid to leave via the exit. The liquid flows through the compartment from the entrance to the exit such that an aerosol is produced. A distance between two adjacent pillars is D and the longitudinal aisle has a width W. The D and the W are specifically configured such that the aerosol has a predetermined MMAD. In certain embodiments, the distance D and the width W are specifically configured to effectively deliver aerosolized drug to patient's lung. To achieve the foregoing, the aerosol must have an MMAD value less than 5.5 um and preferably between 4 um to 5.5 um. Further, for aerosol having MMAD less than 5.5 um, the spray duration is preferred to be approximately 1.6 seconds. Said combination increases the effectiveness of fine particles to be delivered into specific lung regions of a user, thus resulting in a more desirable treatment result. In certain embodiments, the microstructured passage module and the components thereof are specifically configured and arranged such that liquid medicament having certain characteristics can be aerosolized to have predetermined and consistent MMAD and spray duration, under certain liquid medicament conditions. The formulation of liquid medicament contains active pharmaceutical ingredients, stabilizer and preservatives. The active pharmaceutical ingredient may be selected singly or in combination from the group of betamimetics, anticholinergics, antiallergics, antihistamines, and/or steroids. Moreover, the liquid medicament is ethanol-free and may possess certain range of characteristics, such as viscosity and surface tension.

### BRIEF DESCRIPTION OF THE DRAWINGS

One or more embodiments are illustrated by way of example, and not by limitation, in the figures of the accompanying drawings, wherein elements are having the same reference numeral designations represent like elements throughout. The drawings are not to scale, unless otherwise disclosed.
FIG. 1 is a cross section view of an exemplary aerosolizer according to the prior art.
FIG. 2 is a cross section view of another exemplary aerosolizer according to the present disclosure.
FIG. 3A-3B show an exemplary passage module 1 in accordance with some embodiments of the present disclosure.
FIGs. 4A-4C are cross-section views of the microstructured passage module in accordance with some embodiments of the present disclosure.

The drawings are only schematic and are non-limiting. In the drawings, the size of some of the elements may be exaggerated and not drawn on scale for illustrative purposes. The dimensions and the relative dimensions do not necessarily correspond to actual reductions to practice of the invention. Any reference signs in the claims shall not be construed as limiting the scope. Like reference symbols in the various drawings indicate like elements.

### DETAILED DESCRIPTION OF THE DISCLOSURE

The making and using of the embodiments of the disclosure are discussed in detail below. It should be appreciated, however, that the embodiments provide many applicable inventive concepts that can be embodied in a wide variety of specific contexts. The specific embodiments discussed are merely illustrative of specific ways to make and use the embodiments, and do not limit the scope of the disclosure.

Throughout the various views and illustrative embodiments, like reference numerals are used to designate like elements. Reference will now be made in detail to exemplary embodiments illustrated in the accompanying drawings. Wherever possible, the same reference numbers are used in the drawings and the description to refer to the same or like parts. In the drawings, the shape and thickness may be exaggerated for clarity and convenience. This description will be directed in particular to elements forming part of, or cooperating more directly with, an apparatus in accordance with the present disclosure. It is to be understood that elements not specifically shown or described may take various forms.

### Definition

It will be understood that when an element is referred to as being "on" another element, it can be directly on the other element or intervening elements may be present. In contrast, when an element is referred to as being "directly on" another element, there are no intervening elements present.

It will be understood that singular forms "a", "an" and "the" are intended to include the plural forms as well, unless the context clearly indicates otherwise. Furthermore, relative terms, such as "bottom" and "top," may be used herein to describe one element's relationship to other elements as illustrated in the Figures.

It will be understood that elements described as "under" or "below" other elements would then be oriented "over" or "above" the other elements. The exemplary terms "under" or "below" can, therefore, encompass both an orientation of over and under.

The term "about," as used herein, when referring to a measurable value such as an amount, a temporal duration, aerosol measurements, and the like, is meant to encompass variations of ±10% and more preferably ±5% from the specified value, as such variations are appropriate to achieve the intened purpose of the presnet disclosure.

Unless otherwise defined, all terms (including technical and scientific terms) used herein have the same meaning as commonly understood by one of ordinary skill in the art to which this disclosure belongs. It will be further understood that terms; such as those defined in commonly used dictionaries, should be interpreted as having a meaning that is consistent with their meaning in the context of the relevant art and the present disclosure, and will not be interpreted in an idealized or overly formal sense unless expressly so defined herein.

### Detailed Description

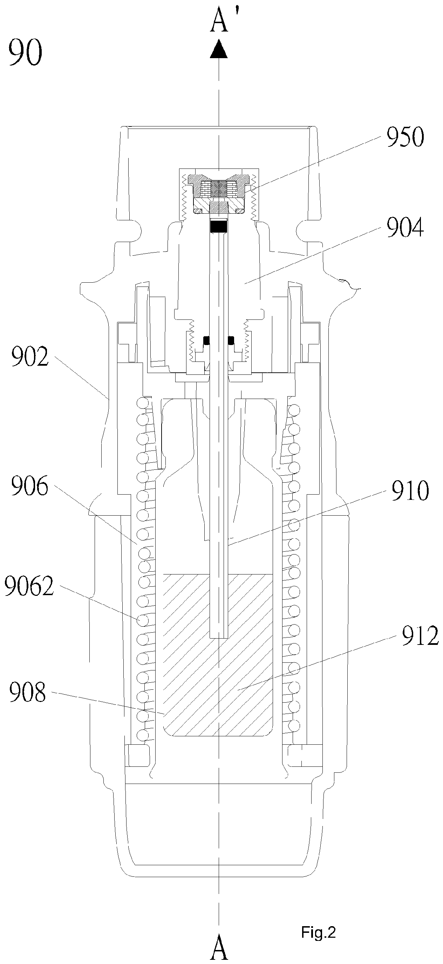

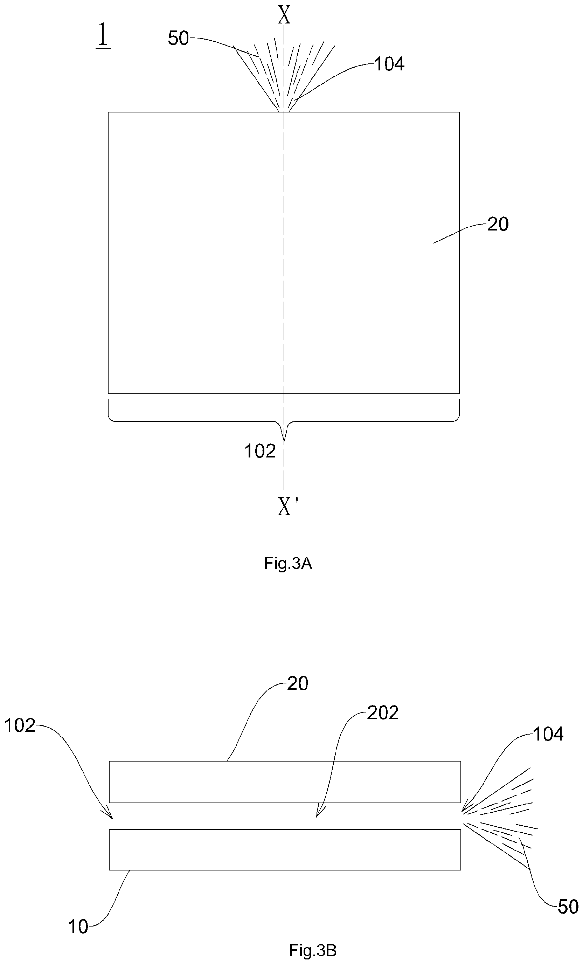

FIG. 2 is a cross-sectional view of an exemplary aerosolizer according to the present disclosure. Here, the aerosolizer 90 includes a housing 902 with a pump chamber 904 and a spring chamber 906. A biasing element 9062, such as a spring, is coupled to the housing 902, and more particularly is mounted in the spring chamber 906. The spring chamber 906 also holds a storage container 908 where liquid medicament 912 is stored. Such liquid medicament 912 can be drawn from the storage container 908 via a tube 910 in response to a pre-actuation of the aerosolizer 90. Particularly, prior to actuation, the housing 902 is rotated. The spring 9062 is adapted to respond to such rotation by tensioning. Correspondingly, the liquid medicament 912 is drawn from the storage container 908 into the pump chamber 904, ready to be aerosolized. The aerosolization process starts when the aerosolizer 90 is actuated. When actuated, a release mechanism (not shown) is triggered and the spring 9062 is released from the tensioned state to the untensioned state. Such operation results in a force pushing the liquid medicament 912 through a transfusion apparatus 950, where a microstructured passage module 1 (e.g., a nozzle) resides (see FIG. 3A), at the pump chamber 904. In other words, the liquid medicament 912 passes through the microstructured passage module 1 for aerosolization. The microstructured passage module 1 is specifically configured such that aerosol having desired particle size in a controlled and precise delivery manner can be produced. Consequently, aerosolized liquid medicament exits the transfusion apparatus 950 and then out of the aerosolizer 90 for patient inhalation. An exemplary liquid medicament contains respirable formulation. Here, the liquid medicament may be an aqueous solution. In preferred embodiments, the liquid medicament is ethanol-free. In other words, the liquid medicament contains no ethanol. More details of the liquid medicament will be discussed later. Moreover, in preferred embodiments, the liquid medicament contains no propellant (exemplary propellants include chlorofluorocarbon or hydrofluoroalkane propellants). Propellants are the driving source for atomized aerosol containing drugs for what's commonly known as pressurized metered dose inhalers (MDI). However, propellants may have adverse impact to the environment. Thus, it is desirable that an aerosolizer is operable without propellants, as disclosed in the present disclosure.

The microstructured passage module 1 is the pivotal component of the aerosolizer 90 where liquid medicament can be broken down into aerosol having fine particles/droplet. The microstructured passage module 1 of the aerosolizer 90 is a component having a microstructured filtering and guiding system, which consists of a plurality of microscale elements and a plurality of passages defined by such microscale elements. When pressured liquid medicament travels into the microstructured passage module 1 at a high speed, the microscale elements will partially block the flowing medicament and parse it into small particles. Furthermore, the configuration of the microscale elements and the passages therebetween will increase flow resistance, thereby reduce the liquid flow speed.

Furthermore, to increase effective aerosol deposition in lung, an ideal aerosol should have certain ranges of MMAD and spray duration. For example, the MMAD should be less than 5.5 um, and the spray duration is between about 1.2 and about 1.6 seconds. In some preferred embodiments, the MMAD is between about 4 um and 6 um; and the spray duration is between about 1.2 and about 1.6 seconds, and more preferably between about 1.4 and about 1.6 seconds. An aerosol having MMAD between about 4 um and 6 um is desirable for inhalation therapy. An aerosol having MMAD higher than such range makes it harder to reach the patient's lung. For example, the aerosol is more likely to be deposited at the throat. On the other hand, an MMAD lower than such range increases the chance of undesired aerosol dissemination. As a result, not enough aerosol reaches the patient's lung, and the therapy is considered ineffective. For spray duration, if that of the aerosol falls out the foregoing range, the inhalation efficiency of the patient will be affected. The chance of clogging or residue may increase, thereby affecting treatment. For example, undesired spray duration may negatively affect the amount of aerosolized medicament a patient inhales at a given time. The present disclosure provides a passage module 1 to better achieve the foregoing MMAD and spray duration. More details of the resulting aerosol will be discussed later.

FIGs. 3A-3B show an exemplary passage module 1 1 in accordance with some embodiments of the present disclosure.

FIG. 3A is a top view of the passage module 1 1 in accordance with some embodiments of the present disclosure. The passage module 1 1 includes a cover 20 and a plate (blocked by the cover 20, not shown). Together, the foregoing forms a compartment to accommodate a filtering structure. Liquid (not shown) enters the compartment via the entrance 102 and leaves in aerosol form via the exit 104. The filtering structure thereof ensures that the resulting aerosol 50 possesses certain characteristics suitable for human inhalation therapy. For example, the aerosol 50 possesses certain MMAD and spray duration as disclosed herein.

FIB. 3B is a sectional view of the passage module 1 1 along the dotted line X-X' as shown in FIG. 3A. Here, the plate 10 and the cover 20 in combination define the entrance 102 for the liquid (not shown) and the exit 104. Moreover, the plate 10 and the cover 20 form a compartment 202. The compartment 202 encompasses the filtering structure (omitted for clarity), which is designed to guide the liquid direction or change a flow speed thereof. The filtering structure may be in contact with both the plate 10 and the cover 20, or not. An example of the filtering structure may be protrusions, pillars, walls or the combination thereof protruding from the plate 10. With the configuration of the filtering structure, the resulting aerosol 50 possesses the desired MMAD and spray duration disclosed herein.

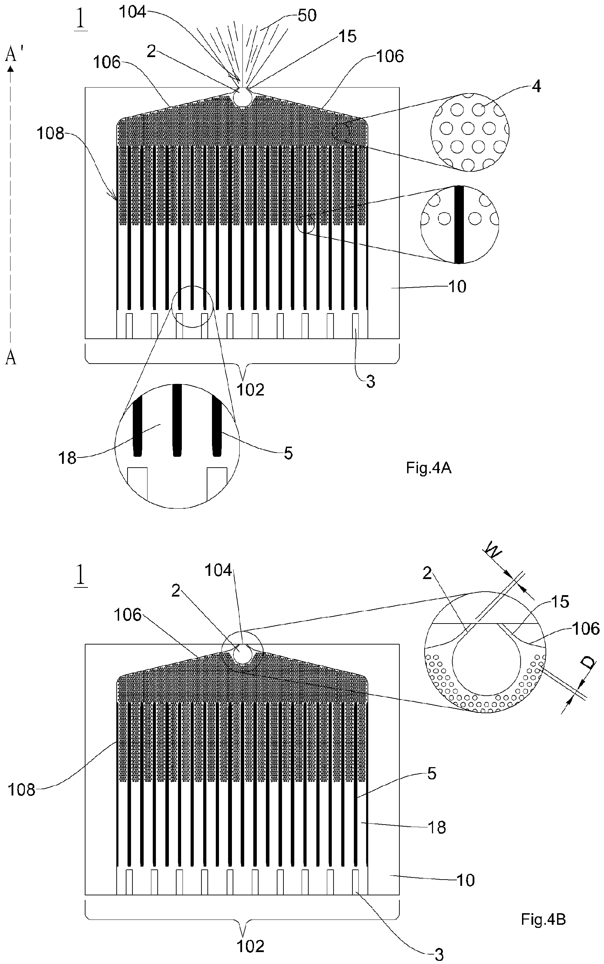

FIGs. 4A-4C are cross-section views of the passage module 1 1 in accordance with some embodiments of the present disclosure.

Referring to FIG. 4A, a microstructured passage module 1 is disclosed. The microstructured passage module 1 includes a plate 10, which can be made from silicon and is about 2.5 mm in width, about 2 mm in length and about 700 um in depth. The plate 10 is overlaid by a glass cover (not shown), which is about 2.5 mm in width, about 2 mm in length and about 675 um in depth. The dimension of the plate 10 and the cover substantially correspond to each other, thus defining a compartment. Moreover, the plate 10 and the cover (not shown) are specifically arranged such that combined together, an entrance 102 and an exit 104 are defined at opposite ends. Between the entrance 102 and the exit 104 are two sidewalls 108, and the distance between the sidewalls 108 is the width of the plate 10. Liquid medicament (not shown) enters the compartment via the entrance 102 at one end. The resulting aerosol 50 leaves the compartment via the exit 104 at the opposite end. The entrance 102 has a width about 2 mm, which is wider that the exit 104. Liquid medicament in the compartment flows along in the general direction from the entrance 102 to the exit 104. A flow direction of the liquid medicament in the passage module 1, which is substantially perpendicular to the entrance 102, is defined by the direction from A to A'. At least some of the liquid medicament will flow along the inclined walls 106 of the passage module 1, causing liquid flows to collide against each other, preferably at about 90°. As a result, aerosol 50 is created.

The plate 10 may further include several components, such as a column 2, spacers 3, pillars 4 and walls 5. The arrangement of pillars 4, the spacers 3 and the walls 5 constitute a filtering structure for the microstructured passage module 1. The spacers 3, walls 5, pillars 4, and column 2 are adapted to project from the plate 10 in the direction transversely to the liquid flow direction In some embodiments, the spacers 3 are configured and arranged in a row proximate to the entrance 102, and a distance between two adjacent spacers 3 is about two times wider than the width of the passage 18. A cross-section of each spacer 3 is rectangular and the dimension of each spacer 3 is about 50 um width and about 200 um long. Generally, the spacers 3 is used as a preliminary filter for the liquid medicament entering the compartment and for dividing the liquid flow into separate passages 18.

In one embodiment, these components may be formed as integral parts of the plate 10 by etching the microstructured passage module 1. In certain embodiments, a depth of about 5-6 um of the plate 10 is etched so as to form such integral components. A depth may have a manufacturing tolerance about 1um. Note that the manufacturing method of the plate 10 is not so limited. The plate 10 may be manufactured by other means known in the relevant art, such as molding, welding or printing. Further characteristic and the configuration of the integral components are further described below.

With reference to FIG. 4B, a column 2, adapted to protrude from the plate 10, is disposed proximate to the exit 104. The column 2 is sphere-like, having a diameterabout 150 um. The column 2 is configured to substantially block the exit 104 to the extent that liquid may only flow to the exit 104 via two aisles 15 formed between the column 2 and the inclined walls 106. The aisles 15 have a least some portions thereof extending continuously and longitudinally. In other words, a part of the inclined wall 106 and the corresponding section of the column 2 may be parallel to each other. The foregoing configuration directs liquid to flow in a manner that would collide with each other, i.e., along two opposite aisles 15. In other words, the microstructured passage module 1 can also be understood to have two exits for the purpose of aerosolization. Opposite liquid jets exiting the two aisles 15 collide into each other at a location external to the passage module 1 but proximate to the exit 104, thereby forming aerosol 50. The column 2 is dimensioned such that each aisle 15 has a width W between about 6.7 um and about 8.3 um, preferably the width W is between about 7 um and about 8 um. Note that a manufacturing tolerance may exist for the distance D and the width W which is about ± 0.3 um. In certain embodiments, the width W is the distance between the inclined wall 106 and the column 2, measurement thereof is shown in FIG. 4B.

Referring to both FIGs. 4A and 4B, the plate 10 further includes walls 5 disposed across its entire width. The filtering structure of the present invention may further include such walls 5, which are longitudinal and parallel to each other in the liquid flow direction A to A'. Between each parallel wall 5 is a passage 18 for the liquid medicament to flow. The liquid flow via the plurality of passages in the direction A to A'. The dimension of such passage is about 77 um wide. The wall may have a general dimension of about 22 um wide.

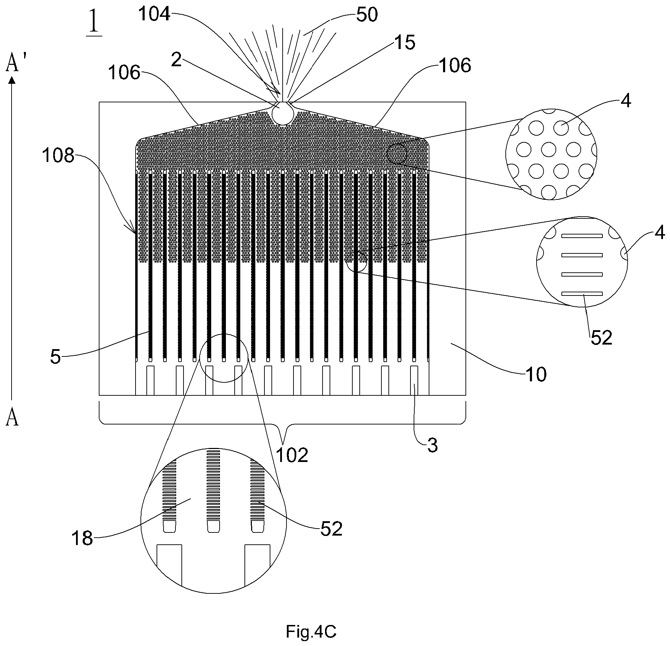

In some embodiments, for the unfiltered liquid medicament entering the microstructured passage module 1, the space between two walls 5 are used as filters. For example, any particle with size larger than the width of the passage 18 will be blocked and therefore filtered. The walls 5 may further guide the direction of the liquid flow, such that liquids flow along the direction A-A' more uniformly. Accordingly, turbulences may be reduced. In some embodiments, as shown in FIG. 4C, the walls 5 are not continuous. For example, a plurality of protrusions 52 are adapted to line up to form the walls 5. Particularly, there is spacing between each adjacent protrusions that form the walls 5. The result might be that liquid flow in each passage becomes communicative with each other as liquid can also flow traversely through the spacing between the protrusions. It is important to note that all technical features pertaining to walls 5 throughout this disclosure is applicable to both continuous and un-continuous walls. In other embodiments, there are no walls 5 and only the pillars 4 serve the filtering function.

As shown in FIG. 4A to 4C, each pillar 4 is circular in shape and evenly distributed. The above configuration forms a symmetrically-patterned filtering structure. As a result, symmetrical liquid flows with respect to the walls 5 and the pillars 4 are generated so as to reduce chance of turbulences within the compartment, which might affect the aerosolization result. The pillars 4 are microscaled elements that project from the plate 10 with a height of about 5-6 um. There is a distance D between each two adjacent pillars, and the distance D is between about 6.7 um and 8.3 um, preferably the distance D is between about 7 um and 8 um. The distribution of pillars 4 may serve to filter the liquid into finer particles or increase flow resistance against the liquid medicament. As a result, the liquid flow speed in the compartment will reduce. In yet some other embodiments, the plate 10 includes both walls 5 and pillars 4. However, the pillars 4 are not present within the aisles 15.

As shown in FIGs. 4A to 4C, the walls 5 start from the entrance 102 and extend towards the exit 104. The walls 5 may or may not extend past the intersection of the sidewall 108 and the inclined wall 106. Moreover, the walls 5 may not start from the entrance 102. In one example, the walls 5 start from a distance from the entrance 102. As for the pillars 4, they occupy at least section of the passages 18. Moreover, the pillars 4 occupy such part of the plate proximate to the exit 104. In embodiments where there is no wall or if the wall is un-continuous, the pillars are evenly distributed over the plate 10. By term "occupy" means that the pillars 4 are present at that vicinity of the plate 10, but do not completely block the flowing of the liquid. In some embodiments, it can be said that the plate includes a first zone and a second zone, and the first zone is closer to the entrance 102 than the second zone. Moreover, in some embodiment, the passages 18 are adapted to be in the first zone, and there is no wall 5 in the second zone. The pillars 4 occupy at least the second zone, and part of the first zone but not entirely.

Attention is now directed to Table 1 below. Table 1 shows a comparison of droplet size known as Mass Median Aerodynamics Diameter (MMAD) measured by Next Generation Impactor (NGI) [Reference: USP 36 (601) Aerosols, Nasal Sprays, Metered-Dose Inhalers, AND Dry Powder Inhalers for aqueous solution. ] In the present disclosure, the distance D and the width W are specifically configured for a pressurized aqueous liquid. The resulting aerosol has certain predetermined MMAD and spray duration.

**Table 1**

| Width W (um) | Distance D (um) | MMAD (um) | FPF (%) | Spray Duration (s) | Spray Velocity (m/s) |
|---|---|---|---|---|---|
| 8 | 8 | 4.4 | 42.8 | 1.25 | 174.7 |
| 7 | 8 | 4.7 | 36.5 | 1.51 | 170.7 |
| 8 | 7 | 4.5 | 41.0 | 1.56 | 169.9 |
| 7 | 7 | 4.5 | 39.4 | 1.57 | 169.8 |

The resulting (n=3) shown in Table 1, aerosol 50 has an MMAD less than about 5.5um, and more preferably between about 4 um and 5.5 um. Moreover, the spray duration thereof is less than 1.6 seconds, particular between about 1.2 and 1.6 seconds and more preferably between about 1.4 and 1.6 seconds. Correspondingly, the spray velocity of the aerosol 50 exiting the exit 104 is between about 169 m/s and 175 m/s. Table 1 further shows a comparison of fine particle fraction (FPF) less than 5 micron for a pressurized aqueous solution. In one embodiment, the fraction of the droplets which are less than 5 micron in size is less than 50% and preferably between 35 % to 45 %.

To achieve the foregoing, the distance D and the width W needs to be specifically configured. In some embodiments, the width W is between about 7 um and 8 um and in combination with the distance D is between about 7 um and 8 um. Preferably, one of the width W and the distance D is less than 8 um, and/or another of the width W and the distance D is larger than 7 um. This is beneficial to generate a MMAD less than about 5.5um and spray duration about 1.5 to 1.6 second. The forgoing leads to a desired particle size and soft mist for delivering drug to the lung of patient.

In other words, patients are capable of inhaling a fixed amount of aerosol having ideal particle size in every operation of the aerosolizer. However, the present disclosure should not be limited to the textual description. That is, any combination of the width W and the distance D within the range specified in Table 1 is within the scope of the present disclosure. In addition, the foregoing is capable of help producing aerosol having desirable MMAD and spray duration disclosed herein.

However, the liquid possesses certain characteristics and the selection thereof relates to the operation and desired result of the aerosolizer. Specifically, the aerosolizer delivers a less than 20ul of liquid solution via an at least 50 bar of pressure source to generate a therapeutically effective propellant-free aerosol. To be considered as therapeutically effective, the aerosol must possess the characteristic disclosed herein. To achieve the foregoing, the liquid itself and the environment thereof must be controlled.

In certain embodiments, the formulation of the liquid contains no propellant gases. Furthermore, the formulation of liquid contains active pharmaceutical ingredients, stabilizer and preservatives. The active pharmaceutical ingredient may be selected singly or in combination from the group of betamimetics, anticholinergics, antiallergics, antihistamines, and/or steroids. For example, the active ingredient is selected singly or in combination form Albuterol Sulfate, Ipratropium Bromide, Tiotropium, Olodaterol, Budesonide, Formoterol, Fenoterol etc. The active pharmaceutical ingredient desirably has a concentration of 0.001 to 2g/100 ml in a solution; A suitable stabilizer may be EDTA (ethylenediamine tetraacetic acid)) having a concentration of 0.001 to 1 mg/ml in a solution, particularly about less than 0.5 mg/ml and preferably about less than 0.25 mg/ml; A suitable preservative may be Benzalkonium Chloride. Moreover, the pH value of the formulation solution is adjusted to a specific range, and the formulation solution may include citric acid, and/or hydrochloric acid. In certain preferred embodiments, the ingredients of the liquid may be tiotropium bromide (or the like) of 0.22-0.23 mg/ml, Benzalkonium (or the like) of 0.08mg/ml-0.12 mg/ml and EDTA (or the like) 0.08-0.12 of mg/ml. Moreover, the pH value is between 2.7-3.1. The acidic pH value may be used to stabilize the formation and achieve the delivery of desired dose level. Moreover, in a preferred embodiment, the liquid has a low viscosity, about 0.00088 Pa.s (0.88 cP) at room temperature. The surface tension of the liquid is between about 43 mN/m and 48 mN/m. In another embodiment, liquid is aerosolized to form a propellant-free aerosol for administering to the lungs of the patient.

As shown in FIG. 2, the liquid is stored in the storage container 908 so as to be later operated within the aerosolizer 90. It is essential that the liquid does not contain any ingredients or undesirable drug characteristics, which may damage or interact with the aerosolizer 90 or the storage container 908. For example, the liquid may be non-ethanolic aqueous solution so that it is stable when stored in the container. Further, such effective quantity of active pharmaceutical ingredient and stabilizer has a desired concentration to avoid device damage or corrosion. For example, if EDTA is used, its concentration within the formulation solution need to be optimized. A high concentration of the EDTA may increase the chance of crystallization formation in the liquid channel of the nozzle, thus causing clogging or blockage.

In addition to the foregoing, the combination of the specific structural design of the microstructured passage module 1 and the selection of liquid formation enables the aerosolizer to produce aerosol having predetermined MMAD and spray duration under an extended range of temperature. Attention is now directed to Table 2 below.

**Table 2**

| Temperature (Celsius) | Width W (um) | Distance D (um) | MMAD (um) | FPF (%) | Spray Duration (s) |
|---|---|---|---|---|---|
| 25 | 7 | 8 | 4.7 | 36.49 | 1.51 |
| 4 | 7 | 8 | 5.2 | 29.25 | 1.47 |

Table 2 shows the effect of different working temperatures for the specifically configured microstructure passage module 1 disclosed herein. It was found that the aerosolizer (n=3) can be operated at working temperature of about 4 to25 degree Celsius. In one example, the storage container holding the drug is stored in a refrigerator, giving it a 4 degrees Celsius environment before the operation of the aerosolizer. As shown by Table 2, the present disclosure enables the aerosolizer to produce aerosol having similar characteristics at 4 degrees and 25 degrees Celsius. In other words, the present disclosure presents a specifically configured microstructured passage module 1 such that desirable aerosol can be produced in a stringent condition. Patients benefit from the foregoing because their aerosol inhalation treatment may be administered under more diversified circumstances. Further, in response to the extended operable temperature range, the aerosolizer becomes suitable for liquid medicament having certain liquid viscosity. In some embodiments, the viscosity of the drug solution is adjusted to about 0.0005 to 0.003 Pa.s (0.5 cP to 3 cP). In certain preferred embodiments, the viscosity ranges from about 0.0008 to 0.0016 Pa.s ( 0.8 cP to 1.6 cP). Note that higher viscosity may affect the average particle size of the aerosol and aerosol spray duration, and it's preferred to keep the viscosity low. Moreover, the configuration of the microstructured passage module 1 of the present disclosure enables it suitable for liquid medicament having certain surface tension. In some embodiments, the surface tension of the drug solution is between about 20 to70 mN/m and preferably between about 25 to 50 mN/m. The lower surface tension may provide a better spreadability of the drug. As a result, aerosol deposition on the lung surface may be improved. This will increase the effectiveness of the drug and thus the inhalation treatment.

Thus, with the above desired liquid formation. the preferred microstructured passage module 1 has the width W between about 6.7 um and 8.3 um, the distance D is between about 6.7 um and 8.3 um during the viscosity range of 0.0005 to 0.003 Pa.s ( 0.5 to 3 cP) (working temperature about 4~25 degree Celsius), resulting an MMAD of less than about 5.5 um and preferably between 4 to 5.5um, a spraying duration of less than 1.6 second, and preferably between 1.4 to 1.6 second, and a fraction of the droplets which are less than 5 micron in size less than 50% and preferably between 25 % to 40 %. Under this setting, the aerosol inhalation treatment is the most effective.

In other words, the present disclosure presents a specifically configured microstructured passage module 1 such that desirable aerosol can be produced in a stringent environmental condition. Patients benefit from the foregoing because their aerosol inhalation treatment may be administered under more diversified circumstances.

Overall, the present disclosure provides a microstructured passage module 1 easier to manufacture because the design and arrangement microscaled components thereof are less complicated. The resulting device can deliver a more accurate doze of aerosol, having desired MMAD and spray duration, in each operation of the aerosolizer.

### LISTING OF ELEMENTS

Passage module 1
Column 2
Spacer 3
Pillar 4
Plate 10
Entrance 102
Exit 104
Inclined wall 106
Sidewall 108
Aisle 15
Passage 18
Aerosol 50
Liquid medicament 912
Protrusion 52
Wall 5
Cover 20
Aerosolizer 90
Housing 902
Pump chamber 904
Spring chamber 906
Biasing element 9062, 962
Spring 9062
Storage container 908, 961
Tube 910, 966
Transfusion apparatus 950
Nozzle 963
Upper housing 964
Lower housing 965
Liquid flow direction A-A'

## Claims

1. A microstructured passage module (1) for an aerosol generator, comprising:
a plate (10) overlaid by a cover (20) thus forming a compartment (202), and the plate and the cover in combination define an entrance (102) and an exit (104) for the compartment, a liquid flow direction is defined by liquid flowing from the entrance to the exit of said compartment;
a plurality of walls (5) along the liquid flow direction and parallel to each other over an entire width of the plate so as to define a plurality of passages threrebetween;
a plurality of pillars (4) protruding from the plate and a distance between two adjacent pillars is D; and
a column (2) protruding from the plate, adapted to be proximate to and substantially blocks the exit to the extent that aisles (15) are formed between the column and the exit for the liquid to flow through, and the aisle has a width W,
wherein the liquid flows through the compartment (202) in the liquid flow direction such that an aerosol having a predetermined MMAD (Mass Median Aerodynamic Diameter) is achieved,
wherein the width W is between 6,7 µm and 8,3 µm, and the distance D is between 6,7 µm and 8,3 µm.

2. The microstructured passage module according to Claim 1, wherein at least one of the width W and the distance D is less than 8 µm.

3. The microstructured passage module according to Claim 2, wherein another of the width W and the distance D is larger than 7 µm.

4. The microstructured passage module according to Claim 1, wherein the predetermined MMAD is less than 5,5 µm; and/or
wherein the aerosol generated further has a spray duration between 1.2 and 1.6 seconds

5. The microstructured passage module according to Claim 1, wherein the fraction of the droplets which are less than 5 micron in size is less than 50%; and
wherein the fraction of the droplets which are less than 5 micron in size is preferably between 35%to 45%.

6. The microstructured passage module according to Claim 1, wherein a cross-section of the pillar (4) is circular.

7. The microstructured passage module according to Claim 6, wherein the pillars (4) are evenly distributed.

8. The microstructured passage module according to Claim 1, wherein the liquid is ethanol-free; and/or
wherein viscosity of the liquid is between 0.5 to 3 cP.

9. An aerosol generator, comprising:
a housing (902);
a pump chamber (904);
a spring chamber (906) having a storage container (908) storing a liquid medicament (912);
a transfusion apparatus (950) having the microstructured passage module (1) according to any one of claims 1 to 8 configured to aerosolize the liquid medicament;
a filtering structure that is configured on the plate (10) and includes the walls (5) and the pillars (4);
wherein the filtering structure is configured to increase a flow resistance thereof, thus an aerosol having a MMAD (Mass Median Aerodynamic Diameter) of less than 5,5 µm is achieved, and
wherein the liquid medicament includes active pharmaceutical ingredients, stabilizer and preservatives.

10. The aerosol generator according to Claim 9, wherein the width W is between 7 µm and 8 µm, and/or
wherein the liquid medicament is alcohol free.

11. The aerosol generator according to Claim 9, wherein the liquid medicament has a viscosity less than 3 cP.

12. The aerosol generator according to Claim 11, wherein the liquid medicament has a viscosity between 0.8 cP to 1.6 cP.

13. The aerosol generator according to Claim 9, wherein the active pharmaceutical ingredients are selected singly or in combination from the group of betamimetics, anticholinergics, antiallergics, and antihistamines.

14. The aerosol generator according to Claim 13, wherein the stabilizer is EDTA and has a concentration less than 0.25 mg/ml

15. The aerosol generator according to Claim 9, wherein the liquid medicament is aerosolized to form a propellant-free aerosol for administering to the lungs of the patient.

## Patentansprüche

1. Mikrostrukturiertes Durchgangsmodul (1) für einen Aerosolerzeuger, umfassend:
eine Platte (10), die von einer Abdeckung (20) überlagert ist und so ein Fach (202) bildet, wobei die Platte und die Abdeckung in Kombination einen Eingang (102) und einen Ausgang (104) für das Fach definieren, wobei eine Flüssigkeitsströmungsrichtung durch eine vom Eingang zum Ausgang des Fachs fließende Flüssigkeit definiert ist;
eine Mehrzahl von Wänden (5) entlang der Flüssigkeitsströmungsrichtung und parallel zueinander über eine gesamte Breite der Platte, um eine Mehrzahl von Durchgängen dazwischen zu definieren;
eine Mehrzahl von Säulen (4), die von der Platte hervorstehen, wobei eine Distanz zwischen zwei benachbarten Säulen D ist; und
eine von der Platte hervorstehende Spalte (2), die so ausgelegt ist, dass sie sich in der Nähe des Ausgangs befindet und diesen im Wesentlichen in dem Maße blockiert, dass zwischen der Spalte und dem Ausgang Gänge (15) gebildet werden, durch die die Flüssigkeit fließt, und wobei der Gang eine Breite W hat,
wobei die Flüssigkeit durch das Fach (202) in der Flüssigkeitsströmungsrichtung fließt, so dass ein Aerosol mit einem vorbestimmten MMAD (medianer massebezogener aerodynamischer Durchmesser) erreicht wird,
wobei die Breite W zwischen 6,7 µm und 8,3 µm beträgt und die Distanz D zwischen 6,7 µm und 8,3 µm beträgt.

2. Mikrostrukturiertes Durchgangsmodul nach Anspruch 1, wobei zumindest eines von der Breite W und der Distanz D weniger als 8 µm beträgt.

3. Mikrostrukturiertes Durchgangsmodul nach Anspruch 2, wobei ein anderes von der Breite W und der Distanz D größer als 7 µm ist.

4. Mikrostrukturiertes Durchgangsmodul nach Anspruch 1, wobei der vorbestimmte MMAD weniger als 5,5 µm beträgt, und/oder
wobei das erzeugte Aerosol ferner eine Sprühdauer zwischen 1,2 und 1,6 Sekunden aufweist.

5. Mikrostrukturiertes Durchgangsmodul nach Anspruch 1, wobei der Anteil der Tropfen, die weniger als 5 µm groß sind, weniger als 50% beträgt.

6. Mikrostrukturiertes Durchgangsmodul nach Anspruch 1, wobei ein Querschnitt der Säule (4) kreisförmig ist.

7. Mikrostrukturiertes Durchgangsmodul nach Anspruch 6, wobei die Säulen (4) gleichmäßig verteilt sind.

8. Mikrostrukturiertes Durchgangsmodul nach Anspruch 1, wobei die Flüssigkeit ethanolfrei ist; und/oder
wobei eine Viskosität der Flüssigkeit zwischen 0,5 und 5 cP beträgt.

9. Aerosolerzeuger, umfassend:
ein Gehäuse (902);
eine Pumpenkammer (904);
eine Federkammer (906), die einen Speicherbehälter (908) aufweist, der ein flüssiges Medikament (912) speichert;
eine Transfusionsvorrichtung (950), die das mikrostrukturierte Durchgangsmodul (1) nach einem der Ansprüche 1 bis 8 aufweist, das dazu konfiguriert ist, das flüssige Medikament zu aerosolieren;
eine Filterungsstruktur, die auf der Platte (10) konfiguriert ist und die Wände (5) und die Säulen (4) enthält;
wobei die Filterungsstruktur dazu konfiguriert ist, einen Strömungswiderstand davon zu erhöhen, wodurch ein Aerosol erhalten wird, das einen MMAD (medianer massebezogener aerodynamischer Durchmesser) von weniger als 5,5 µm aufweist, und
wobei das flüssige Medikament aktive pharmazeutische Inhaltsstoffe, Stabilisatoren und Konservierungsmittel enthält.

10. Aerosolerzeuger nach Anspruch 9, wobei die Breite W zwischen 7 µm und 8 µm beträgt, und/oder
wobei das flüssige Medikament alkoholfrei ist.

11. Aerosolerzeuger nach Anspruch 9, wobei das flüssige Medikament eine Viskosität von weniger als 3 cP aufweist.

12. Aerosolerzeuger nach Anspruch 11, wobei das flüssige Medikament eine Viskosität zwischen 0,8 cP und 1,6 cP aufweist.

13. Aerosolerzeuger nach Anspruch 9, wobei die aktiven pharmazeutischen Inhaltsstoffe einzeln oder in Kombination ausgewählt sind aus der Gruppe der Betamimetika, Anticholinergika, Antiallergika und Antihistaminika.

14. Aerosolerzeuger nach Anspruch 13, wobei der Stabilisator EDTA ist und eine Konzentration von weniger als 0,25 mg/ml aufweist.

15. Aerosolerzeuger nach Anspruch 9, wobei das flüssige Medikament aerosoliert wird, um treibgasfreies Aerosol zur Verabreichung in die Lungen des Patienten zu bilden.

## Revendications

1. Un module de passage microstructuré (1) pour un générateur d'aérosol, comprenant :
une plaque (10) recouverte par un couvercle (20) formant ainsi un compartiment (202), et la plaque et le couvercle en combinaison définissent une entrée (102) et une sortie (104) pour le compartiment, une direction d'écoulement de liquide est définie par un liquide s'écoulant de l'entrée à la sortie dudit compartiment ;
une pluralité de parois (5) le long de la direction d'écoulement du liquide et parallèles entre elles sur toute une largeur de la plaque de manière à définir une pluralité de passages entre elles ;
une pluralité de piliers (4) dépassant de la plaque et une distance entre deux piliers adjacents est D ; et
une colonne (2) faisant saillie depuis la plaque, adaptée pour être proche de la sortie et obstruant sensiblement celle-ci, à un point tel que des allées (15) sont formées entre la colonne et la sortie pour que le liquide s'écoule à travers elles, et l'allée a une largeur W,
le liquide s'écoulant à travers le compartiment (202) dans la direction d'écoulement du liquide de sorte qu'un aérosol ayant un MMAD (diamètre aérodynamique médian en masse) prédéterminé est obtenu,
la largeur W étant comprise entre 6,7 µm et 8,3 µm, et la distance D étant comprise entre 6,7 µm et 8,3 µm.

2. Le module de passage microstructuré selon la revendication 1, dans lequel au moins l'une parmi la largeur W et la distance D est inférieure à 8 µm.

3. Le module de passage microstructuré selon la revendication 2, dans lequel une autre parmi la largeur W et la distance D est supérieure à 7 µm.

4. Le module de passage microstructuré selon la revendication 1, dans lequel le MMAD prédéterminé est inférieur à 5,5 µm ; et/ou
dans lequel l'aérosol généré a en outre une durée de pulvérisation comprise entre 1,2 et 1,6 seconde.

5. Le module de passage microstructuré selon la revendication 1, dans lequel la fraction des gouttelettes de taille inférieure à 5 microns est inférieure à 50 % ; et
dans lequel la fraction des gouttelettes qui ont une taille inférieure à 5 microns est de préférence comprise entre 35 % et 45 %.

6. Le module de passage microstructuré selon la revendication 1, dans lequel une section transversale du pilier (4) est circulaire.

7. Le module de passage microstructuré selon la revendication 6, dans lequel les piliers (4) sont répartis de façon régulière.

8. Le module de passage microstructuré selon la revendication 1, dans lequel le liquide est exempt d'éthanol ; et/ou
dans lequel la viscosité du liquide est comprise entre 0,5 et 3 cP.

9. Un générateur d'aérosol, comprenant :
un boîtier (902) ;
une chambre de pompe (904) ;
une chambre de ressort (906) ayant un récipient de stockage (908) stockant un médicament liquide (912) ;
un appareil de transfusion (950) ayant le module de passage microstructuré (1) selon l'une quelconque des revendications 1 à 8 configuré pour aérosoliser le médicament liquide ;
une structure filtrante configurée sur la plaque (10) et comprenant les parois (5) et les piliers (4) ;
la structure filtrante étant configurée pour augmenter sa résistance à l'écoulement, ainsi un aérosol ayant un MMAD (diamètre aérodynamique médian en masse) inférieur à 5,5 µm est obtenu et
le médicament liquide comprenant des ingrédients pharmaceutiques actifs, un stabilisant et des conservateurs.

10. Le générateur d'aérosol selon la revendication 9, dans lequel la largeur W est comprise entre 7 µm et 8 µm et/ou dans lequel le médicament liquide est exempt d'alcool.

11. Le générateur d'aérosol selon la revendication 9, dans lequel le médicament liquide a une viscosité inférieure à 3 cP.

12. Le générateur d'aérosol selon la revendication 11, dans lequel le médicament liquide a une viscosité comprise entre 0,8 cP et 1,6 cP.

13. Le générateur d'aérosol selon la revendication 9, dans lequel les ingrédients pharmaceutiques actifs sont choisis seuls ou en combinaison dans le groupe des bêtamimétiques, des anticholinergiques, des antiallergiques et des antihistaminiques.

14. Le générateur d'aérosol selon la revendication 13, dans lequel le stabilisant est l'EDTA et a une concentration inférieure à 0,25 mg/ml.

15. Le générateur d'aérosol selon la revendication 9, dans lequel le médicament liquide est mis en aérosol pour former un aérosol sans propulseur à administrer aux poumons du patient.
